(19) Europäisches Patentamt
European Patent Office
Office européen des brevets

(11) Publication number: **0 069 442**
**B1**

(12) **EUROPEAN PATENT SPECIFICATION**

(45) Date of publication of patent specification: **20.02.85**

(51) Int. Cl.$^4$: **C 07 D 249/08**, A 61 K 31/41

(21) Application number: **82302075.5**

(22) Date of filing: **22.04.82**

(54) **Antifungal agents, processes for their preparation, and pharmaceutical compositions containing them.**

(30) Priority: **06.06.81 GB 8117379**
**17.10.81 GB 8131370**
**04.03.82 GB 8206329**

(43) Date of publication of application:
**12.01.83 Bulletin 83/02**

(45) Publication of the grant of the patent:
**20.02.85 Bulletin 85/08**

(84) Designated Contracting States:
**AT BE CH DE FR GB IT LI LU NL SE**

(58) References cited:
**EP-A-0 044 605**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE CH DE FR IT LI LU NL SE AT**

(72) Inventor: **Richardson, Kenneth**
**48 St. Stephens Hill**
**Canterbury Kent (GB)**

(74) Representative: **Wood, David John et al**
**Pfizer Limited Ramsgate Road**
**Sandwich Kent CT13 9NJ (GB)**

**EP 0 069 442 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a novel bis-triazole derivative which has antifungal activity and is useful in the treatment of fungal infections in animals, including humans.

According to the invention, there is provided the compound of the formula:—

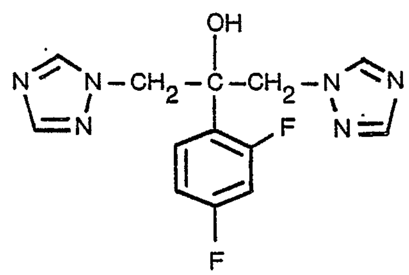

(I)

and its pharmaceutically acceptable salts.

British Patent Application No. 2078719A (ICI) published 13.01.82, and European application No. 0044605 (ICI) published 27.01.82, disclose compounds of the general formula:

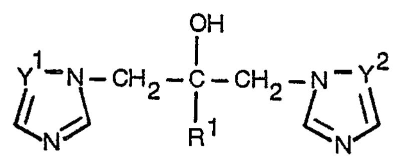

where $R^1$ is an optionally substituted-alkyl, -cycloalkyl (e.g. cyclopentyl or cyclohexyl), -aryl (e.g. phenyl) or -aralkyl (e.g. benzyl) group; and $Y^1$ and $Y^2$ are =CH— or =N—; and salts or metal complexes, ethers or esters thereof.

These compounds are stated to be useful as fungicides and plant growth regulants. They are also stated to be active against fungus diseases of humans.

In particular, these applications disclose in Example 2 and claim 7, 1,3-bis-(1,2,4-triazol-1-yl)-2-(2,4-dichlorophenyl)-propan-2-ol. This compound has the formula:—

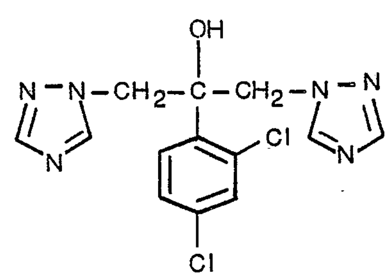

The 2,4-difluorophenyl compound of the present invention is not specifically disclosed in these ICI applications, nor is "2,4-difluorophenyl" specifically named as a preferred example of "$R^1$".

This 2,4-dichlorophenyl compound is teratogenic whilst, quite unexpectedly, the 2,4-difluorophenyl analogue of the present invention is not teratogenic.

In addition, the corresponding 2-, 3- and 4-chlorophenyl and 4-bromophenyl analogues are teratogenic.

This was determined by the following teratology studies:—

TERATOLOGY STUDIES

Following insemination, female rats (Crl: COBS—CD(SD)BR, Charles River Breeding Colony, France) were randomly assigned into groups of five animals. The test compounds were administered daily as a suspension in 0.1% aqueous methylcellulose by gastric intubation for ten consecutive days from day 6 to day 15 post insemination.

The animals were sacrificed on day 20 post-insemination and the number of dead foetuses together with the number, sex and weight of viable foetuses were recorded. All the foetuses were examined for external, buccal and visceral abnormalities.

The compounds tested were as follows:—

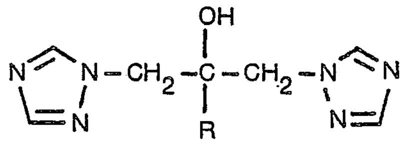

where R = 2,4-dichlorophenyl, 2-, 3- and 4-chlorophenyl, 4-bromophenyl or 2,4-difluorophenyl.

All foetuses from animals treated with the compound in which R = 2,4-dichlorophenyl at 20 mg/kg body weight showed external malformations, in particular cleft palates. Examination of visceral and skeletal features revealed that this compound was teratogenic at doses as low as 1 mg/kg. e.g. presence of microphthalmia, increased incidence of dilatation of the ureters and renal pelves, delay in ossification of some bones, and increased incidence of a 14th pair of ribs.

Also, the compound in which R = 4-chlorophenyl was extremely embryotoxic at 20 mg/kg, whilst the compound in which R = 2-chlorophenyl produced external abnormalities (cleft palate) at this dose. These compounds are specifically disclosed as "Compounds 1 and 9," respectively in Table 1 of the ICI applications. In addition, the compounds in which R = 3-chlorophenyl and R = 4-bromophenyl, which are claimed but not specifically disclosed in the ICI applications, also produced the same external abnormalities at 20 mg/kg. The latter compound was also embryotoxic at this dose.

The specific compound claimed in our invention, i.e. the compound in which R = 2,4-difluorophenyl, when administered to pregnant rats under identical conditions, produced most surprisingly, no external malformations. Internal examination of foetuses from these animals revealed no significant visceral or skeletal abnormalities.

The foetotoxicity (teratogenicity and/or embryotoxicity) of the said compounds in which R is other than 2,4-difluorophenyl is a major disadvantage should it be desired to use them for human mycoses.

The invention also provides a pharmaceutical composition comprising a compound of the formula (I) or a pharmaceutically acceptable salt thereof, together with a pharmaceutically acceptable diluent or carrier. The composition is preferably for human use and in tablet, capsule, injectable or ointment form.

The invention further provides a compound of the formula (I) or a pharmaceutically acceptable salt thereof, for use in treating fungal infections in humans.

The compound of the formula (I) can be obtained by a number of different processes. In one process according to the invention it is obtained by the reaction of the oxirane of the formula:

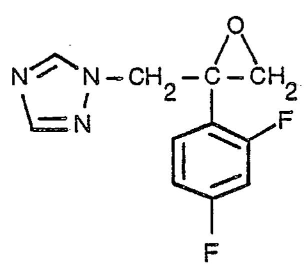

(II)

with 1,2,4-triazole, preferably in the presence of a base.

The preferred bases are potassium carbonate and sodium hydride.

In a typical procedure, 1,2,4-triazole, the oxirane (II) and anhydrous potassium carbonate are reacted together in a suitable solvent, e.g. dry dimethylformamide, preferably with heating to 50°—120°C to accelerate the reaction, which is generally complete in 8 hours or less. The product can be isolated and purified by conventional methods.

The triazole starting material can also be used in its acid addition salt form (e.g. as the mesylate salt), in which case excess base should be used.

The oxirane (II) can be obtained by conventional methods, typically from the corresponding ketone (III):

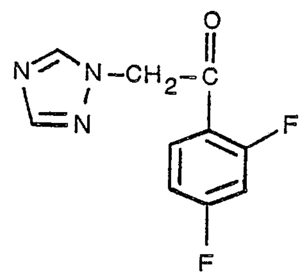

(III)

by reacting it with dimethyloxosulphonium methylide prepared from trimethylsulphoxonium iodide and cetrimide/sodium hydroxide.

This reaction is typically achieved as follows. The ketone (III), trimethylsulphoxonium iodide and cetrimide are stirred vigorously in a mixture of toluene and aqueous sodium hydroxide solution for a few hours at up to about 100°C. The oxirane product can then be isolated by conventional procedures.

The ketone (III) can be prepared by conventional methods, e.g. as follows:—

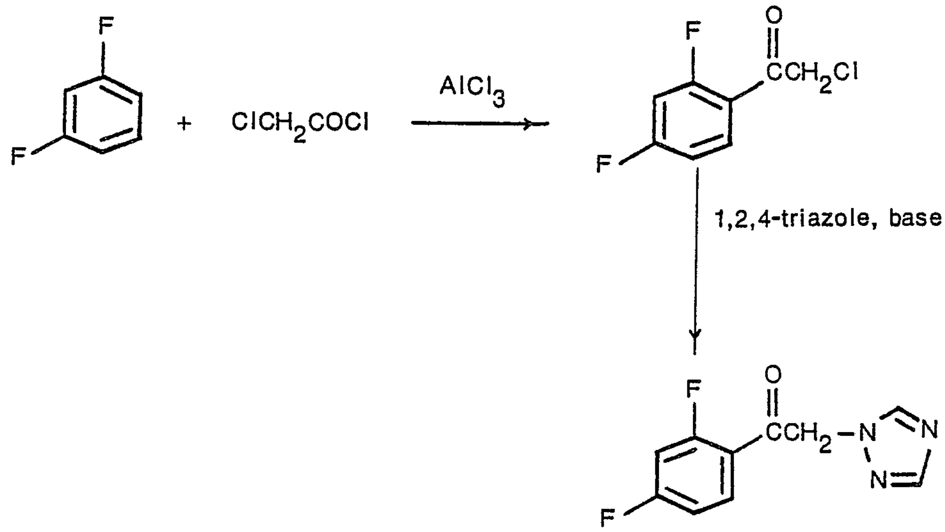


In addition the invention provides a method for making the compound of the formula (I), as follows:—

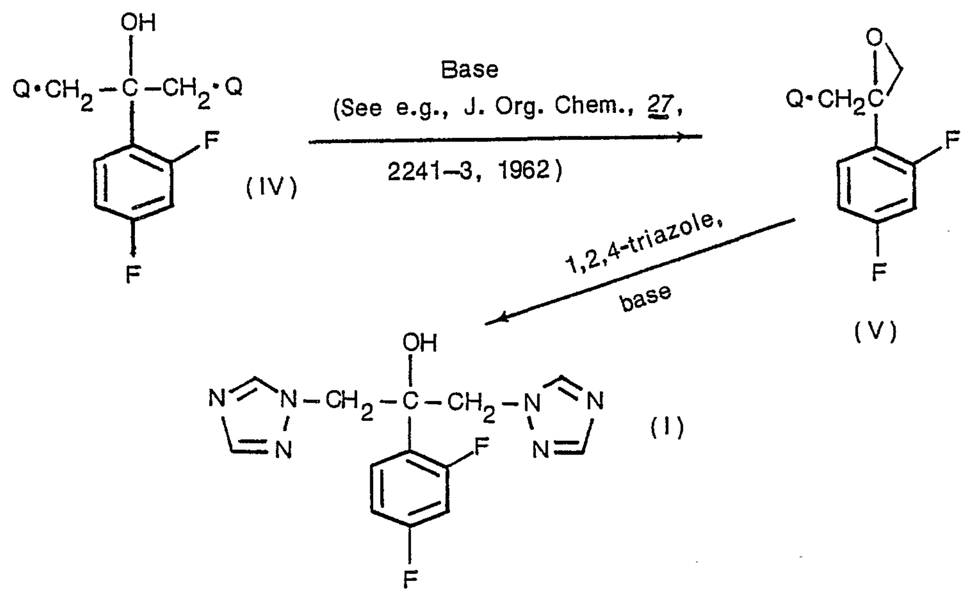


(IV) (V) (I)

(Q is a facile leaving group, preferably Cl, Br or I).

It is believed that the intermediate oxirane of the formula (V), which can be isolated if desired, is formed in this reaction.

In a typical reaction, the compound (IV) and 1,2,4-triazole are heated together, e.g. at up to 120°C, in a suitable solvent, e.g. dry N,N-dimethylformamide or tetrahydrofuran, for up to about 24 hours, preferably in the presence of a base, e.g. potassium carbonate. Generally an excess of the triazole and base are used. The product (I) can then be isolated and purified by conventional procedures.

In both methods described above, the product (I) will generally be contaminated with the isomer in which one of the triazole rings is attached to the adjacent $CH_2$ via the 4-position, but this unwanted isomer can be removed by chromatography on e.g. silica gel, or by recrystallisation.

The starting material of the formula (IV) can be obtained by conventional procedures, e.g.

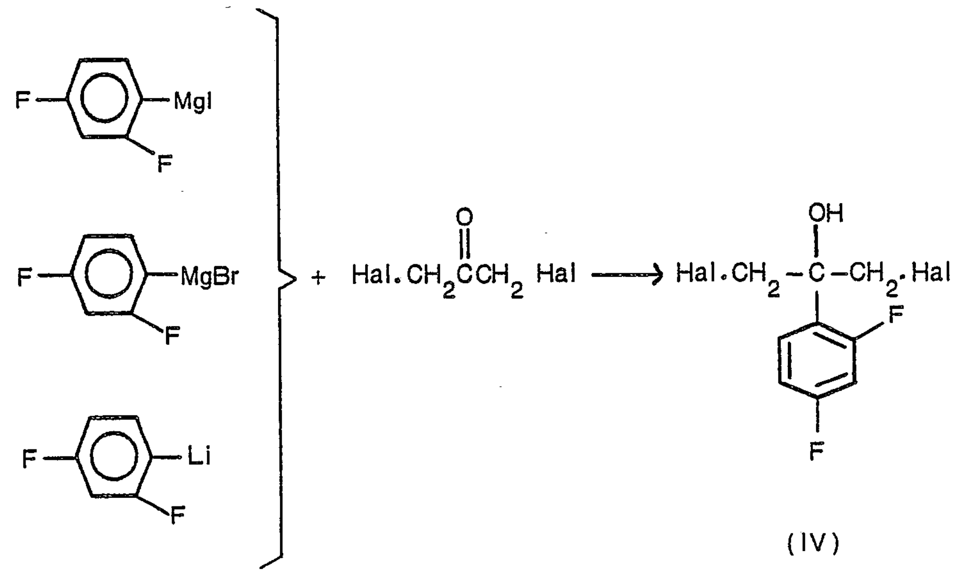

(IV)

The preferred pharmaceutically acceptable salts are the acid addition salts. Pharmaceutically acceptable acid addition salts of the compounds of the formula (I) are those formed from strong acids which form non-toxic acid addition salts containing pharmaceutically-acceptable anions, such as the hydrochloride, hydrobromide and sulphate.

The salts may be obtained by conventional procedures, e.g. by mixing solutions containing equimolar amounts of the free base and desired acid, and the required salt is collected by filtration, if insoluble, or by evaporation of the solvent.

The compound of the formula (I) and its pharmaceutically acceptable salts are very active anti-fungal agents, useful in combating fungal infections in animals, including humans. For example they are useful in treating topical fungal infections in man caused by, among other organisms, species of *Candida, Trichophyton, Microsporum,* or *Epidermophyton*, or in mucosal infections caused by *Candida albicans* (e.g. thrush and vaginal candidiasis). They may also be used in the treatment of systemic fungal infections caused by, for example, *Candida albicans, Cryptococcus neoformans, Aspergillus fumigatus, Coccidioides, Paracoccidioides, Histoplasma* or *Blastomyces*.

The *in vitro* evaluation of the anti-fungal activity of the compound (I) can be performed by determining the minimum inhibitory concentration (m.i.c.) which is the concentration of the test compound in a suitable medium at which growth of the particular micro-organism fails to occur. In practice, a series of agar plates, each having the test compound incorporated at a particular concentration are inoculated with a standard culture of, for example *Candida albicans* and each plate is then incubated for 48 hours at 37°C. The plates are then examined for the presence or absence or growth of the fungus and the appropriate m.i.c. value is noted. Other micro-organisms used in such tests can include *Cryptococcus neoformans, Aspergillus fumigatus, Trichophyton* spp, *Microsporum* spp, *Epidermophyton floccosum, Coccidioides immitis,* and *Torulopsis glabrata.*

The *in vivo* evaluation of the compound can be carried out at a series of dose levels by intraperitoneal or intravenous injection or by oral administration, to mice which are inoculated with a strain of *Candida albicans*. Untreated mice die within 48 hours and the dose level at which the compound provides 50% protection against the lethal effect of the infection is noted. The compound (I) gives at least 50% protection at below 0.5 mg/kg. (p.o. or i.v.).

For human use, the anti-fungal compound of the formula (I) (or salt thereof) can be administered alone, but will generally be administered in admixture with a pharmaceutical carrier selected with regard to the intended route of administration and standard pharmaceutical practice. For example, it may be administered orally in the form of a tablet containing such excipients as starch or lactose, or in a capsule or ovule either alone or in admixture with excipients, or in the form of an elixir or suspension containing a flavouring or colouring agent. It may be injected parenterally, for example, intravenously, intramuscularly or subcutaneously. For parenteral administration, it is best used in the form of a sterile aqueous solution which may contain other substances, for example, enough salts or glucose to make the solution isotonic.

For oral and parenteral administration to human patients, it is expected that the daily dosage level of the anti-fungal compound of the formula (I) will be from 0.1 to 5 mg/kg (in divided doses) when administered by either the oral or parenteral route. Thus tablets or capsules of the compounds can be expected to contain from 5 mg to 0.5 g of active compound for administration singly or two or more at a time as appropriate. The physician in any event will determine the actual dosage which will be most suitable for an individual patient and it will vary with the age, weight and response of the particular patient. The above dosages are exemplary of the average case. There can, of course, be individual instances where

higher or lower dosage ranges are merited, and such are within the scope of this invention.

Alternatively, the anti-fungal compound of formula (I) may be administered in the form of a suppository or pessary, or it may be applied topically in the form of a lotion, solution, ointment or dusting powder. For example, it may be incorporated into a ointment consisting of an aqueous emulsion of polyethylene glycols or liquid paraffin; or it may be incorporated, at a concentration between 1 and 10%, into an ointment consisting of a white wax or white soft paraffin base together with such stabilizers and preservatives as may be required.

The following Examples illustrate the invention:—

Example 1

*Preparation of 2-(2,4-Difluorophenyl)-1,3-bis(1H-1,2,4-triazol-1-yl)propan-2-ol*

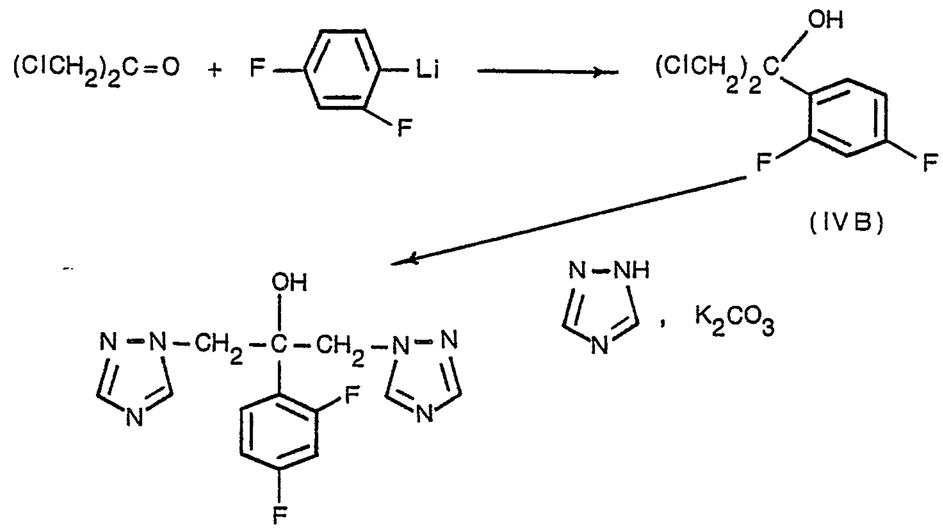

(i) A solution of 1-bromo-2,4-difluorobenzene (0.96 g, 5 mM) in diethyl ether (10 ml) was stirred at −78°C and a solution of *n*-butyl lithium (1.55 M, 3.23 ml; 5 mM) in hexane was added over three minutes. After the addition was complete, the mixture was stirred for a further 10 minutes and then a solution of 1,3-dichloroacetone (0.63 g, 5 mM) in diethyl ether (10 ml) was added dropwise over three minutes. After stirring for a further 30 minutes at −78°C, a solution of acetic acid (0.33 g) in diethyl ether (5 ml) was added at 0°C, followed by water (10 ml). The organic layer was separated and the aqueous layer was washed once with diethyl ether. The combined ether extracts were dried ($MgSO_4$) and evaporated to give a pale yellow oil which was dissolved in dimethyl formamide (20 ml). This DMF solution contained the intermediate (IVB).

(ii) 1,2,4-Triazole (1.72 g, 25 mM) and anhydrous potassium carbonate (2.07 g, 15 mM) were added to the solution prepared in (i) and the mixture was heated at 70°C for 18 hours. The reaction mixture was cooled and poured into water (100 ml). The aqueous mixture was extracted twice with ethyl acetate. The combined organic extracts were dried ($MgSO_4$) and evaporated to give a gum. The gum was chromatographed on silica (270—400 Mesh), eluting with 3% methanol in methylene chloride, to give the title compound as a white solid, 0.40 g (26% based on dichloroacetone), m.p. (after crystallisation from ethyl acetate/hexane), 138—140°C.

Analysis %:—

| | | | |
|---|---|---|---|
| Calculated for $C_{13}H_{12}F_2N_6O$: | C,50.98; | H,3.95; | N,27.44. |
| Found: | C,51.33; | H,4.05; | N,27.08. |

$^1H$,n.m.r., i.r. and mass spectral data were consistent with the required structure. The chromatographic procedure separated the desired product from the 1-[2-(2,4-difluorophenyl)-2-hydroxy-3-(4H-1,2,4-triazol-4-yl)-propyl]-1H-1,2,4-triazole impurity that was present in the reaction mixture.

Example 2

(A) *Preparation of 2-chloro-2′,4′-difluoroacetophenone*

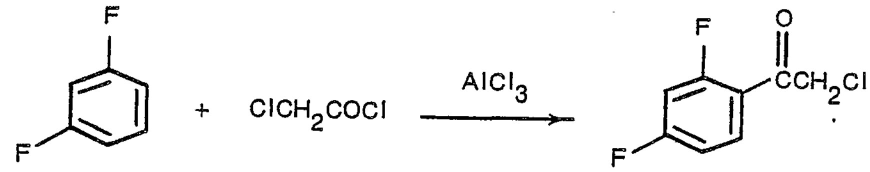


Chloroacetyl chloride (113 g, 1.0 M) was added dropwise to a stirred mixture of 1,3-difluorobenzene (114 g, 1.0 M) and anhydrous aluminium chloride (146.6 g, 1.1 M) at room temperature (20°C). The mixture was stirred for a further five hours at 50°—55°C. Methylene chloride (48.5 ml) was added slowly as the mixture was allowed to cool to room temperature. The methylene chloride layer was separated, washed with water (2 × 320 ml) and the solvent removed by distillation at reduced pressure leaving a pale yellow solid (180 g).

A portion of the crude product (145 g) was crystallised from n-hexane (435 ml) giving the title compound (113 g, 73%) m.pt. 47°—49°C (literature* 46.5°C). IR (KRr) and nmr ($CDCl_3$) were consistent with the desired structure.

* Von D. Ehlers, H. Bercher and A. Grisk,
*J. Prakt, Chem., 315.* 1169 (1973).

(B) *Preparation of 2,4,-difluoro-2-(1H-1,2,4-triazol-1-yl) acetophenone hydrochloride*

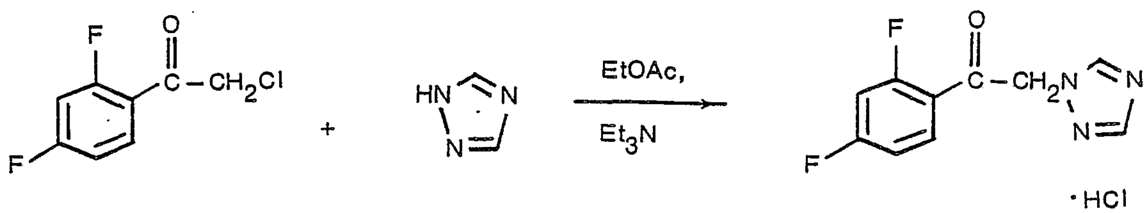


To a mixture of 1,2,4-triazole (30.4 g, 0.44 M) and triethylamine (15.1 g, 0.15 M) in refluxing ethyl acetate (186 ml) was added a solution of 2-chloro-2′,4′-difluoroacetophenone (38.1 g, 0.2 M) in ethyl acetate (80 ml). The mixture was refluxed for six hours then cooled to room temperature and the insolubles were removed by filtration. The filtrate was washed with water (2 × 200 ml) and then the solvent was removed by distillation at reduced pressure. The crude product was dissolved in ethyl acetate (150 ml) then 25% w/v HCl gas in isopropanol was added. The mixture was granulated at 0°C for one hour and then the solid was collected by filtration and dried to give the title compound (21.6 g, 40%), melting point 167°—170°C. IR (KBr) and nmr (DMSO) were consistent with the desired structure.

This intermediate was characterised as the free base, which was prepared by the following technique:—

To a stirred slurry of sodium bicarbonate (16.8 g, 0.2 M) and 1,2,4-triazole (27.6 g, 0.4 M) in refluxing toluene (180 ml) was added a solution of 2-chloro-2′,4′-difluoroacetophenone (38.1 g, 0.2 M) in toluene (45 ml). The mixture was stirred at reflux for three hours and the water formed during the reaction was removed using a Dean and Stark trap. The reaction mixture was cooled to room temperature and then water (180 ml) was added. The toluene layer was separated and the solvent removed by distillation at reduced pressure. The resulting pale brown solid was crystallised from 1:1 ethylacetate:*n*-hexane (70 ml) giving the title compound (3.9 g), melting point 103°—105°C. The IR (KBr) and nmr ($CDCl_3$) were consistent with the desired structure.

Analysis%:—

| | | | |
|---|---|---|---|
| Calculated for $C_{10}H_7F_2N_3O$: | C,53.8; | H,3.16; | N,18.82. |
| Found: | C,53.62; | H,3.15; | N,18.68. |

(C) *Preparation of 1-[2-(2,4-Difluorophenyl)-2,3-epoxypropyl]-1H-1,2,4-triazole methanesulphonate*

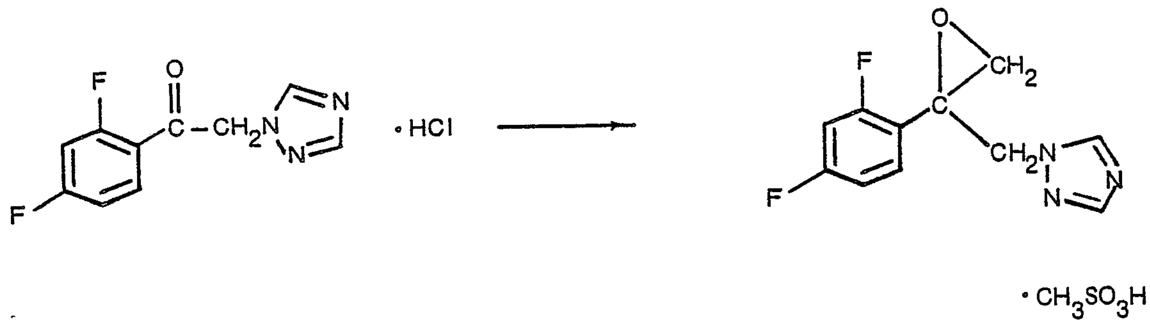

2[1],4[1]-Difluoro-2-(1H-1,2,4-triazol-1-yl)acetophenone hydrochloride (59.6 g, 0.23 M), trimethylsulphoxonium iodide (50.6 g, 0.23 M) and cetrimide (2.1 g) were stirred in a mixture of toluene (370 ml) and 20% w/w aqueous sodium hydroxide at 60° for 3 hours. The toluene layer was separated and concentrated to 110 ml then diluted with ethyl acetate (150 ml). A solution of methanesulphonic acid (16.6 g, 0.172 M) in ethyl acetate (20 ml) was added. More ethyl acetate (100 ml) was added and the mixture was stirred at 0°C for one hour then filtration of the precipitate gave the title compound (43 g, 56%).

20 g of the crude product was dissolved in hot industrial methylated spirits (140 ml) and carbon (2 g) was added. The mixture was filtered and the filtrate was concentrated to 100 ml then the mixture was stirred at 0°C for one hour. Filtration gave the title compound (7.8 g, 39%), melting point 128°—129°C. The IR (KBr) and nmr (DMSO) were consistent with the desired structure.

| | | | |
|---|---|---|---|
| Analysis % | | | |
| Calculated for $C_{12}H_{13}F_2N_3O_4S$: | C, 43.2; | H, 3.9; | N, 12.6. |
| Found: | C, 42.83; | H, 3.92; | N, 12.96. |

(D) *Preparation of 2-(2,4-difluorophenyl)-1,3,bis(1H-1,2,4-triazol-1-yl)-propan-2-ol*

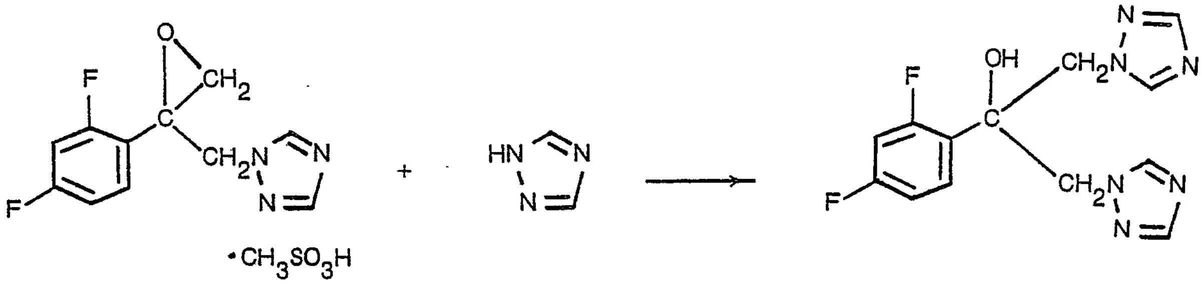

1-[2-(2,4-Difluorophenyl)-2,3-epoxypropyl]-1H-1,2,4-triazole methanesulphonate (6.7 g, 0.02 M), 1,2,4-triazole (2.8 g, 0.04 M) and anhydrous potassium carbonate (9.1 g, 0.066 M) were stirred in dimethylformamide (35 ml) at 90°C for 4½ hours. The mixture was cooled to room temperature then added to water (170 ml). The mixture was extracted with chloroform (2 × 60 ml) and the extracts combined and washed with water (2 × 100 ml). The chloroform solution was dried ($MgSO_4$) and the solvent removed by distillation at reduced pressure leaving the crude product (5.3 g).

The crude product was dissolved in isopropanol (50 ml) and carbon (0.5 g) was added. The mixture was filtered and the filtrate concentrated to 25 ml. The precipitate was collected and dried to give the title compound (2.6 g, 44%) melting point 139°—140°C. The IR (KBr) and nmr (DMSO) were consistent with the desired structure.

| | | | |
|---|---|---|---|
| Analysis:— | | | |
| Calculated for $C_{13}H_{12}F_2N_6O$: | C, 51.0; | H, 3.92; | N, 27.5. |
| Found: | C, 50.85; | H, 3.92; | N, 27.74. |

**Claims for the Contracting States: BE CH DE FR GB IT LI LU NL SE**

1. The compound of the formula:—

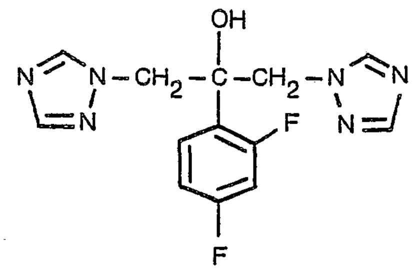

(I)

and its pharmaceutically acceptable salts.

2. A process for preparing a compound of the formula (I) as claimed in claim 1, which comprises reacting an oxirane of the formula:—

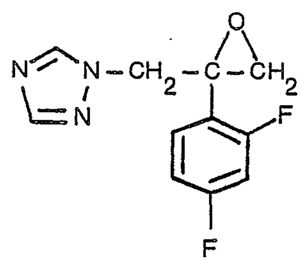

(II)

with 1,2,4-triazole.

3. A process for preparing a compound of the formula (I) as claimed in claim 1, which comprises reacting a compound of the formula:—

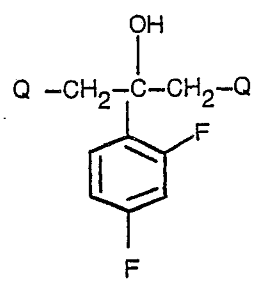

(IV)

where Q is a facile leaving group, with 1,2,4-triazole.

4. A process as claimed in claim 3, where Q is Cl or Br.

5. A process as claimed in claim 2, 3 or 4, which is carried out in the presence of a base.

6. A pharmaceutical composition comprising a compound of the formula (I) as claimed in claim 1 or a pharmaceutically acceptable salt thereof, together with a pharmaceutically acceptable diluent or carrier.

7. A composition as claimed in claim 6 which is for human use and is in tablet, capsule, injectable or ointment form.

8. A compound of the formula (I) as claimed in claim 1 or a pharmaceutically acceptable salt thereof for use in treating a fungal infection in a human being.

**Claims for the Contracting State: AT**

1. A process for preparing a compound of the formula:—

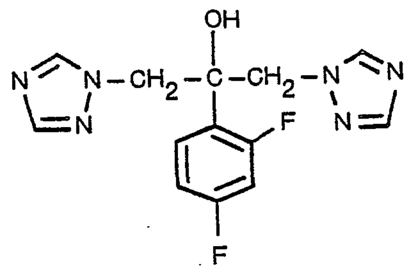

(I)

or a pharmaceutically acceptable salt thereof, which comprises reacting 1,2,4-triazole with a compound of the formula:—

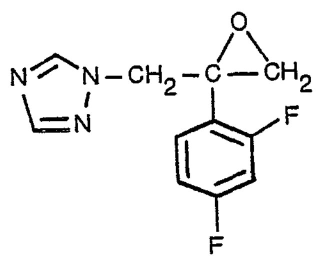

(VI)

followed by, optionally, conversion of the product of the formula (I) into a pharmaceutically acceptable salt.

2. A process for preparing a compound of the formula (I) as defined in claim 1, which comprises reacting a compound of the formula:—

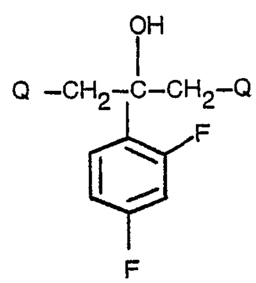

(IV)

where Q is a facile leaving group, with 1,2,4-triazole, followed by, optionally, conversion of the product of the formula (I) into a pharmaceutically acceptable salt.

3. A process as claimed in claim 2, where Q is Cl or Br.

4. A process as claimed in claim 1, 2 or 3, which is carried out in the presence of a base.

**Patentansprüche für die Vertragsstaaten: BE CH DE FR GB IT LI LU NL SE**

1. Verbindung der Formel

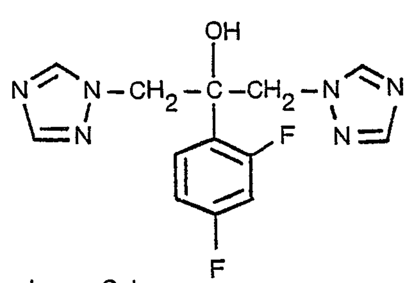

(I)

und ihre pharmazeutisch annehmbaren Salze.

2. Verfahren zur Herstellung einer Verbindung der Formel (I) gemäß Anspruch 1, das die Umsetzung eines Oxirans der Formel

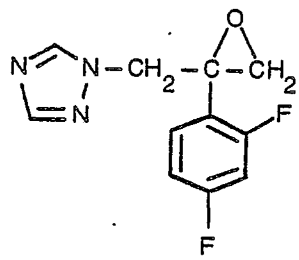

(II)

mit 1,2,4-Triazol umfaßt.

3. Verfahren zur Herstellung einer Verbindung der Formel (I) gemäß Anspruch 1, das die Umsetzung einer Verbindung der Formel

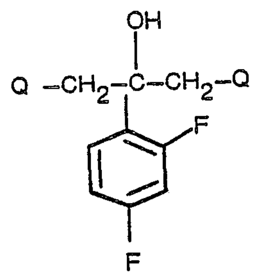

(IV)

worin Q eine leicht austretende Gruppe ist, mit 1,2,4-Triazol umfaßt.

4. Verfahren nach Anspruch 3, worin Q Cl oder Br ist.

5. Verfahren nach Anspruch 2, 3 oder 4, das in Gegenwart einer Base durchgeführt wird.

6. Pharmazeutische Zusammensetzung, umfassend eine Verbindung der Formel (I) gemäß Anspruch 1 oder ein pharmazeutisch annehmbares Salz von dieser, zusammen mit einem pharmazeutisch annehmbaren Verdünnungsmittel oder Träger.

7. Zusammensetzung nach Anspruch 6, die für menschliche Verwendung ist und in Tabletten-, Kapsel-, injizierbarer oder Salbenform ist.

8. Verbindung der Formel (I) gemäß Anspruch 1 oder deren pharmazeutisch annehmbares Salz zur Verwendung bei der Behandlung einer Pilzinfektion in einem Menschen.

**Patentansprüche für den Vertragsstaat: AT**

1. Verfahren zur Herstellung einer Verbindung der Formel

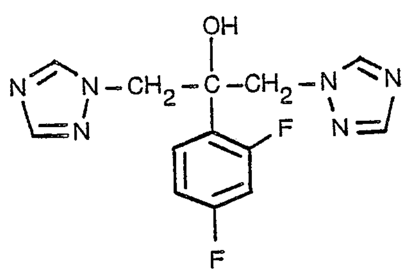

(I)

oder eines pharmazeutisch annehmbaren Salzes hiervon, das die Umsetzung von 1,2,4-Triazol mit einer Verbindung der Formel

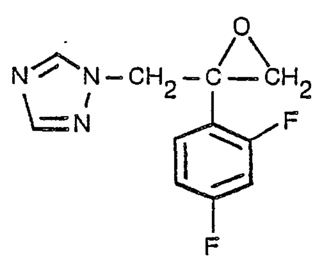

(VI)

gegebenenfalls gefolgt von einer Umwandlung des Produkts der Formel (I) in ein pharmazeutisch annehmbares Salz, umfaßt.

2. Verfahren zur Herstellung einer Verbindung der Formel (I), wie in Anspruch 1 definiert, das die Umsetzung einer Verbindung der Formel

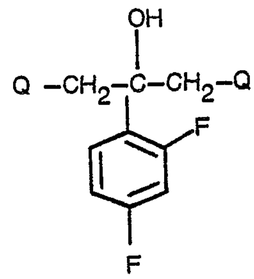

worin Q eine leicht austretende Gruppe ist, mit 1,2,4-Triazol, gegebenenfalls gefolgt von einer Umwandlung des Produkts der Formel (I) in ein pharmazeutisch annehmbares Salz, umfaßt.

3. Verfahren nach Anspruch 2, worin Q Cl oder Br ist.

4. Verfahren nach Anspruch 1, 2 oder 3, das in Gegenwart einer Base durchgeführt wird.

**Revendications pour les Etats contractants: BE CH DE FR GB IT LI LU NL SE**

1. Le composé de formule:

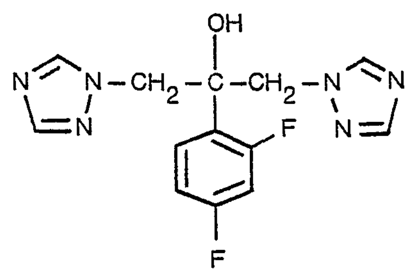

(I)

et ses sels pharmaceutiquement acceptables.

2. Procédé de préparation d'un composé de formule (I) suivant la revendication 1, qui consiste à faire réagir un oxirane de formule:

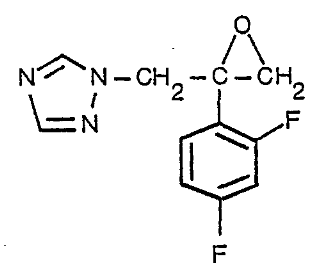

(II)

avec le 1,2,4-triazole.

3. Procédé de préparation d'un composé de formule (I) suivant la revendication 1, qui consiste à faire réagir un composé de formule:

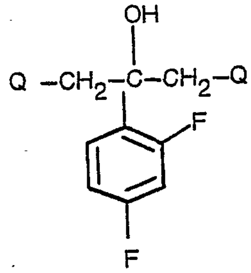

(IV)

dans laquelle Q est un groupe aisément partant, avec le 1,2,4-triazole.

4. Procédé suivant la revendication 3, dans lequel Q représente Cl ou Br.

5. Procédé suivant la revendication 2, 3 ou 4, qui est mis en oeuvre en présence d'une base.

6. Composition pharmaceutique, comprenant un composé de formule (I) suivant la revendication 1 ou un sel pharmaceutiquement acceptable de ce composé, en association avec un diluant ou support pharmaceutiquement acceptable.

7. Composition suivant la revendication 6, à l'usage de l'homme et sous la forme d'un comprimé, d'une capsule, d'une composition injectable ou d'une pommade.

8. Composé de formule (I) suivant la revendication 1 ou un sel pharmaceutiquement acceptable de ce composé, destiné à être utilisé dans le traitement d'une infection fongique chez un être humain.

**Revendications pour l'Etat contractant: AT**

1. Procédé de préparation d'un composé de formule:

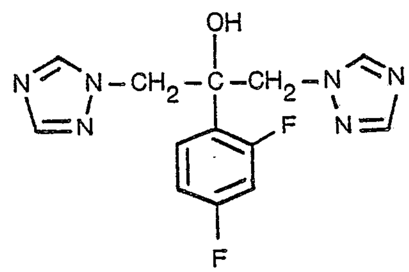

(I)

ou d'un sel pharmaceutiquement acceptable de ce composé, qui consiste à faire réagir le 1,2,4-triazole avec un composé de formule:

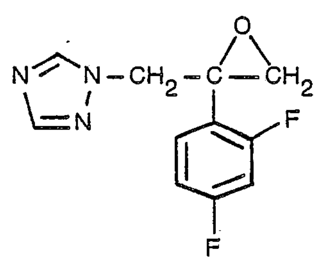

(VI)

la réaction étant suivie, éventuellement, de la transformation du produit de formule (I) en un sel pharmaceutiquement acceptable.

2. Procédé de préparation d'un composé de formule (I) tel que défini dans la revendication 1, qui consiste à faire réagir un composé de formule:

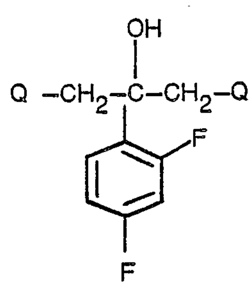

dans laquelle Q est un groupe aisément partant, avec le 1,2,4-triazole, la réaction étant suivie, éventuellement, de la transformation du produit de formule (I) en un sel pharmaceutiquement acceptable.

3. Procédé suivant la revendication 2, dans lequel Q représente Cl ou Br.

4. Procédé suivant la revendication 1, 2 ou 3, qui est mis en oeuvre en présence d'une base.